# EUROPEAN PATENT APPLICATION

(11) **EP 4 596 104 A1**
(43) Date of publication of application: **06.08.2025**
(21) Application number: 23870990.1
(22) Date of filing: 28.09.2023
(51) Int. Cl.: B01J 29/40, B01J 29/48, C01B 39/40

(54) **MFI MOLECULAR SIEVE CATALYST, PREPARATION METHOD THEREFOR AND USE THEREOF**

(30) Priority: 29.09.2022 CN 202211203811
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: TENG, Jiawei, Shanghai 201208 (CN); ZHAO, Guoliang, Shanghai 201208 (CN); JIN, Wenqing, Shanghai 201208 (CN); SHI, Jing, Shanghai 201208 (CN); REN, Liping, Shanghai 201208 (CN); HE, Wanren, Shanghai 201208 (CN)
(74) Representative: karo IP
(86) International application number: PCT/CN2023/122412
(87) International publication number: WO 2024/067765

(57) **Abstract**

Disclosed is an MFI molecular sieve, characterized in that, each unit mass of the MFI molecular sieve comprises coexisting monoclinic crystal form and orthorhombic crystal form of the MFI molecular sieve; and the MFI molecular sieve shows hydroxyl characteristic peaks at 3400-3500 cm⁻¹ in the hydroxyl infrared spectrum thereof, wherein the characteristic peaks have the highest point at a position between 3420-3480 cm⁻¹, and the peak area of the one centering around the highest point and having a half-peak width of 210-240 is in a proportion of 70% or more of the total peak area; and wherein the MFI molecular sieve comprises the elements Si, Al and O, and is free of the element Ti.

## Description

### Technical field

The present disclosure relates to the technical field of catalytic cracking, and particularly relates to a new MFI molecular sieve catalyst and its preparation method, as well as use of the same in increasing production of propylene and ethylene via catalytic cracking of olefins.

### Background

Low-carbon olefins (mainly propylene and ethylene) are important basic organic chemical raw materials. In recent years, the demand for ethylene and propylene has substantially increased due to the increasing demand for polyolefins and alkyl aromatic compounds. At present, most of the propylene produced all over the world comes from by-products of steam cracking and catalytic cracking units. The ratio of propylene to ethylene in steam cracking units is about 0.6. The investment cost for enhancing propylene yield in FCC units in a refinery is high. In this regard, to increase production of propylene, various countries have developed a plurality of alternative methods for producing propylene, such as the butene isomerization (olefin metathesis technology, OMT) process, which increases production of propylene by reacting ethylene with butene; the propane dehydrogenation (PDH) process, which directly uses propane for chemical applications; and the methanol to olefins (MTP/MTO) process, which uses coal-based methanol to produce propylene and ethylene. An important research and development direction for petrochemical companies around the world is new processes for increasing production of propylene and ethylene via catalytic cracking of olefins by using the large amount of C4 and C5 olefins produced by ethylene plants, FCC units and MTO units. Catalysts are the key technology of catalytic processes, wherein their reaction rate, selectivity and stability are closely related to the pore structure and crystalline structure of the catalysts. The catalytic performances and product distributions may be directly affected by post-treatments, element modifications and formation of hydrothermal-resistant molecular sieves with new structures, and the like.

Olefin cracking catalysts are mainly MFI molecular sieves with regular pore channels. They may be prepared by conventional molecular sieve synthesis methods, involving an aging time of 4-48 hours and a crystallization time of 6-120 hours. Such methods are time-consuming and have low process efficiency. When using the obtained catalysts in reactions, due to the side reactions associated with the cracking, such as polymerization, dehydrocyclization and the like, carbon deposits are easily accumulated on the surface of the aluminum silicate molecular sieve, making the catalyst have low catalytic activity, low selectivity for the two olefin products and poor stability. EP0109059A1 discloses a process for cracking C₄-C₁₂ olefins to produce propylene, wherein ZSM 5 or ZSM 11 molecular sieve obtained by a traditional synthesis method is used as the catalyst. US6307117 discloses a process for cracking C₄-C₁₂ olefins to produce propylene and ethylene, wherein the catalyst involves an active component of ZSM-5 molecular sieve obtained by a traditional synthesis method which contains no protonic acid but metals of the Group 1B. CN200910072747.7 introduces a method for preparing MFI molecular sieves, comprising steps of: adding pre-crystallized seeds into a template-free gel system for synthesizing nano molecular sieves, then crystallizing at 160-180°C for 24 hours, and cooling to room temperature, with the product being subjected to centrifuging, filtering, washing, drying, and calcining, to obtain a molecular sieve without impurity crystal phase. The above patents all use traditional molecular sieve synthesis methods, and the resulting olefin cracking catalysts have disadvantages, such as poor product selectivity, poor catalytic stability, and easy coking and deactivating, to various degrees.

### Summary of the invention

The present disclosure is to address the problems of poor stability, low selectivity for the two olefin products and low efficiency in traditional synthesis associated with the current catalysts for producing propylene and ethylene via catalytic cracking of olefins. Provided in the present disclosure are an MFI molecular sieve, a particle catalyst containing the same, an MFI molecular sieve catalyst containing the same, and a preparation method thereof, as well as use of the catalyst in increasing the production of propylene and ethylene via catalytic cracking of olefins. When the new MFI molecular sieve catalyst in accordance with the present disclosure is used for producing propylene and ethylene via catalytic cracking of olefins, it has advantages of good stability and high selectivity for the two olefin products.

In one aspect, provided in the present disclosure is an MFI molecular sieve, characterized in that, each unit mass of the MFI molecular sieve comprises coexisting monoclinic crystal form and orthorhombic crystal form of the MFI molecular sieve; and the MFI molecular sieve shows hydroxyl characteristic peaks at 3400-3500 cm⁻¹ in the hydroxyl infrared spectrum thereof, wherein the characteristic peaks have the highest point at a position between 3420-3480 cm⁻¹, and the peak area of the one centering around the highest point and having a half-peak width of 210-240 accounts for a proportion of 70% or more of the total peak area; and wherein the MFI molecular sieve comprises the elements Si, Al and O, and is free of the element Ti.

For the invention, the infrared spectrum is an absorption spectrum. Accordingly, the characteristic peaks are absorption peaks.

In a further aspect, provided in the present disclosure is a catalyst particle, comprising:
A) the MFI molecular sieve in accordance with the present disclosure;
B) at least two different templates T1 and T2; and
C) a binder.

In a furthermore aspect, provided in the present disclosure is an MFI molecular sieve catalyst, comprising:
a) the MFI molecular sieve in accordance with the present disclosure;
optionally, b) a boron group element R1; and
optionally, c) a nitrogen group element R2.

Accordingly, as an example, provided in one embodiment in accordance with the present disclosure is an MFI molecular sieve catalyst, wherein each unit mass of the catalyst comprises coexisting monoclinic crystal form and orthorhombic crystal form of the MFI molecular sieve. For example, monoclinic crystalline molecular sieve and orthorhombic crystalline molecular sieve may be in a molar ratio of 5-100 (i.e. 5:1-100:1). Preferably, the MFI molecular sieve catalyst may show hydroxyl characteristic peaks at 3400-3500 cm⁻¹ in the hydroxyl infrared spectrum thereof, wherein the characteristic peaks have the highest point at a position between 3420-3480 cm⁻¹, and the peak area of the one centering around the highest point and having a half-peak width of 210-240 accounts for a proportion of 70% or more of the total peak area.

For the invention, based on the specific raw materials and specific preparation process used in the present disclosure, the inventor surprisingly found that, significantly different from simple physical mixing, the monoclinic crystalline molecular sieve and the orthorhombic crystalline molecular sieve in accordance with the present disclosure coexist at molecular level. Accordingly, when each unit mass of the MFI molecular sieve, the particle catalyst or the MFI molecular sieve catalyst in accordance with the present disclosure is analyzed, except for obvious measurement errors, obtained is the coexisting of two crystal forms (the monoclinic crystal form and the orthorhombic crystal form) of the MFI molecular sieve. For the purpose of the invention, the unit mass can be understood and determined as the minimum mass of the powder sample that can be tested by XRD, for example, 500mg, 300mg or 200mg of the powder sample. Without being bound by any known theory, it is believed that the coexisting of the two crystal forms of the MFI molecular sieve obtained in the present disclosure is particularly advantageous for the purpose of the invention, such as the required conversion rate and selectivity.

The inventor further surprisingly found that, the MFI molecular sieve, the particle catalyst or the MFI molecular sieve catalyst in accordance with the present disclosure shows in the hydroxyl infrared spectrum thereof hydroxyl characteristic peaks at 3400-3500 cm⁻¹, each of which has typical broad peak features. In particular, for example, the hydroxyl characteristic peaks have the highest point at a position between 3420-3480 cm⁻¹, and the peak area of the one centering around the highest point and having a half-peak width of 210-240 accounts for a proportion of 70% or more of the total peak area. As commonly understood and used in the art, for the purposes of the present invention, when calculating the peak area for the hydroxyl infrared spectrum, the integrated area is concerned, and the line connecting the two lowest points in the curve of the spectrum is the starting reference line for the integration.

Further, the MFI molecular sieve, the particle catalyst or the MFI molecular sieve catalyst in accordance with the present disclosure may respectively show in the hydroxyl infrared spectrum thereof an absorption peak with the maximum intensity at 3460-3470 cm⁻¹.

Further, the MFI molecular sieve, the particle catalyst or the MFI molecular sieve catalyst in accordance with the present disclosure may respectively comprise monoclinic crystal form and orthorhombic crystal form in a molar ratio of preferably 6:1-50:1, more preferably 8:1-20:1, further more preferably 8:1-15:1.

Further, the MFI molecular sieve, the particle catalyst or the MFI molecular sieve catalyst in accordance with the present disclosure may respectively comprise monoclinic crystal form and orthorhombic crystal form in a molar ratio of, for example, not limited to, 8.1:1, 8.2:1, 8.3:1, 8.4:1, 8.5:1, 8.6:1, 8.7:1, 8.8:1, 8.9:1, 9:1, 9.1:1, 9.2:1, 9.3:1, 9.4:1, 9.5:1, 9.6:1, 9.7:1, 9.8:1, 9.9:1, 10:1, and the like.

Further, the MFI molecular sieve catalyst may have a specific surface area of 200-1000 m²/g, preferably 300-800m²/g, more preferably 400-600 m²/g.

Further, in the MFI molecular sieve catalyst, the molecular sieve is a hydrogen type molecular sieve.

Further, the MFI molecular sieve catalyst may comprise, based on the total weight of the catalyst:
a) the MFI molecular sieve, in an amount of 90-100%, preferably 92-99%;
b) the boron group element R1, in an amount of 0-5%, preferably 0.5-3.0%; and
c) the nitrogen group element R2, in an amount of 0-5%, preferably 0.5-5.0%.

Further, in the MFI molecular sieve, the particle catalyst or the MFI molecular sieve catalyst, the molecular sieve may have a molar ratio of SiO₂/Al₂O₃ of 80-1500, preferably 80-1000.

Further, in the particle catalyst or the MFI molecular sieve catalyst, the boron group element R1 is at least one selected from the group consisting of B and Ga; and/or, the nitrogen group element R2 is at least one selected from the group consisting of N, P, As, Sb and Bi.

Further, the particle catalyst or the MFI molecular sieve catalyst may further comprise a binder. Based on the weight of the catalyst, the binder may be present in an amount of 5% or less, preferably 2% or less, further preferably 0.5% or less.

In a further aspect, provided in the present disclosure is a method for preparing the above-mentioned MFI molecular sieve catalyst, comprising steps of:
Step 1: preparing an MFI molecular sieve raw powder;
Step 2: kneading and shaping the molecular sieve raw powder obtained in Step 1 with a binder, and drying, to obtain catalyst particles;
Step 3: subjecting the catalyst particles obtained in Step 2 to hydrothermal crystallization under an atmosphere of a template T3, and ammonium exchanging, to obtain the MFI molecular sieve catalyst. Step 3, especially the hydrothermal crystallization and ammonium exchanging, helps to obtain the hydrogen-type molecular sieve required by the invention.

In one embodiment of the invention, in the above method for preparing the MFI molecular sieve catalyst, the operation of Step 1 and Step 2 may obtain the particle catalyst in accordance with the present disclosure.

Furthermore, Step 1 of preparing the MFI molecular sieve raw powder may comprise steps of:
preparing a raw gel, by mixing a silicon source, an aluminum source, a template T1, a template T2, water and optionally, the boron group element R1 and/or optionally, the nitrogen group element R2;
crystallizing, washing, and drying.

Further, in the preparing the raw powder of MFI molecular sieve, for the purpose of the invention, the template T1 is preferably an ammonium compound, for example, at least one selected from the group consisting of tetrapropylammonium bromide, tetrapropylammonium hydroxide, tetramethylammonium bromide, tetraethylammonium bromide, tetrabutylammonium bromide, tetramethylammonium hydroxide, tetraethylammonium hydroxide, and tetrabutylammonium hydroxide. Preferably, for the purpose of the invention, the template T2 is a specific template containing an amino group, preferably for example, at least one of phthalimide, taurine, and naphthalene diamine. The introducing T2 and adjusting the ratio of the template T1 to the template T2 may control the ratio of the orthorhombic crystal form to the monoclinic crystal form, leading to the catalyst.

Further, in the preparing the MFI molecular sieve raw powder, the silicon source may be at least one of silica sol, tetraethyl orthosilicate, and silicates; and/or, the aluminum source may be at least one of aluminum sulfate, aluminum isopropoxide, and aluminates.

Further, in the preparing the MFI molecular sieve raw powder, the silicon source, the aluminum source, the templates, the boron group element, the nitrogen group element and water may be in molar ratios of: H₂O/SiO₂=10-500; Si/Al=20-∞; T1/SiO₂=0.01-20; T2/T1=0.01-30, preferably 0.1-20; R1/SiO₂=0-50, preferably, 0.1-30; R2/SiO₂=0-50, preferably 0.1-30.

Further, the preparing the MFI molecular sieve raw powder is operated in a supergravity reactor. The supergravity reactor is a known type of reactor in the art. That kind of reactor is used in the present disclosure and subjected to specific configuration. For example, it may comprise: preparing the raw gel by mixing the silicon source, the aluminum source, the template T1, the template T2, water and optionally, the boron group element R1 and/or optionally, the nitrogen group element R2; sending the mixture into a supergravity aging-crystallizing integrated device for premixing and circulating, then subjecting to direct crystallizing, discharging from the device, and drying, to obtain the MFI molecular sieve raw powder. The crystallizing is once-through crystallizing directly operated after the premixing and circulating in the supergravity aging-crystallizing integrated device. Further, the supergravity aging-crystallizing integrated device may comprise a feeding system, a reaction system, and a discharging system. Further, the raw gel is fed through the feeding system of the supergravity aging-crystallizing integrated device, then subjected to the premixing and circulating and the crystallizing in sequence in the reaction system, and discharging through the discharging system out of the device. The supergravity reactor may have a rotation speed of 0-3000rpm, preferably 1000-2500rpm, 1000-2000rpm. Further, the raw gel may be fed into the supergravity aging-crystallizing integrated device at a feed rate of 10-2000ml/min, preferably 15-300ml/min. The supergravity reactor may adopt a flow rate of 10mL/min-5000mL/min, preferably 20mL/min-1000mL/min, for a time of 0.5-15h. The crystallizing may be operated at a temperature of 80-180°Cfor a time of 5min-24h. Synthesizing the molecular sieve raw powder in the supergravity reactor imparts the catalyst with a higher specific surface area.

Further, in the preparing the MFI molecular sieve raw powder, the washing and the drying follow the crystallizing. The washing may be operated with deionized water. The drying may be operated under conditions of: at a drying temperature of 80-100°C for a drying time of 10-20h.

Further, in the preparing the MFI molecular sieve raw powder, the boron group element R1 and the nitrogen group element R2 may be directly added into the mother liquor for synthesizing the molecular sieve, without the need for impregnation and post-treatment, to obtain the new MFI molecular sieve in one step.

Further, in Step 2, the binder may be a silicon compound, or a mixture of a silicon compound and an aluminum compound. The aluminum compound may be at least one selected from the group consisting of alumina and aluminum sol, and the silicon compound may be at least one selected from the group consisting of white carbon black and silica sol. On the total weight of alumina and silica basis, the binder may be added in an amount of 3%-50%, preferably 5%-40% of the total weight of the molecular sieve raw powder and the binder. For the invention, preferably, the aluminum sol and/or silica sol may involve water as solvent thereof.

Furthermore, in Step 2, the shaping may be operated by any conventional method, such as extruding into strips, and the like. According to the requirements during the shaping, an appropriate amount of water may be added, for example, in the cases wherein the binder is white carbon black, or a mixture of white carbon black and alumina. The drying may be operated under conditions of: at a drying temperature of 80-120°C, preferably 90-100°C, for a drying time of 5-10h, preferably 6-8h.

Further, in Step 3, the catalyst particles obtained in Step 2 may be placed in the atmosphere of the template T3, to be subjected to hydrothermal crystallization at a temperature of 100-200°C, preferably 120-150°C, for a time of 12-180h, preferably 24-32h.

Furthermore, in Step 3, the template T3 may be at least one of aqueous ammonia, ethylamine, ethylenediamine, triethylamine, n-butylamine, hexamethylenediamine, tetrapropylammonium bromide or tetrapropylammonium hydroxide. The atmosphere of the template may be generated by volatilizing an aqueous solution of the template T3 under autogenous pressure in a closed system. The aqueous solution of the template T3 may have a weight concentration of 0.5-40%, preferably 2-30%. The aqueous solution of the template T3 and the catalyst particles may be in a weight ratio of 1:1-5:1.

Further, in Step 3, after hydrothermal crystallization, the obtained product is subjected to washing, drying, and a first calcining. The washing may be operated with deionized water. The drying may be operated under conditions of: at a drying temperature of 80-100°C, preferably 90-100°C, for a drying time of 10-20h, preferably 12-16h. The first calcining may be operated under conditions of: at a calcination temperature of 500-650°C, preferably 530-600°C, for a calcination time of 8-15h, preferably 10-12h.

Further, in Step 3, the ammonium exchanging shall be operated in an aqueous solution of an ammonium salt, wherein the ammonium salt is one or more selected from the group consisting of ammonium chloride, ammonium nitrate, and ammonium sulfate. The aqueous solution of the ammonium salt may comprise the ammonium salt in a weight content of 5%-10%. The ammonium exchanging may be operated at a temperature of 80-90°C and repeated 3-6 times. The product obtained after the ammonium exchanging may be subjected to a second calcining, wherein the second calcining may be operated under conditions of: at a calcination temperature of 500-600°C for a calcination time of 4-8h.

As understood by those skilled in the art, for the MFI molecular sieve catalyst in accordance with the present disclosure, the calcining may remove raw materials such as the templates, and the like. Accordingly, the hydroxyl characteristic peak between 3400 and 3500 cm⁻¹ shown in the hydroxyl infrared spectrum of the catalyst is what the MFI molecular sieve catalyst shows, rather than corresponding to other raw materials such as the templates added during the preparation.

In a further aspect, provided in the present disclosure is use of the MFI molecular sieve catalyst in producing propylene and ethylene via catalytic cracking of olefins.

Further, the producing propylene and ethylene via catalytic cracking of olefins may be operated by: contacting an olefin raw material with the above-mentioned MFI molecular sieve catalyst to react, to obtain the products propylene and ethylene.

Further, it may be operated by using at least one of C4-C6 olefins as the raw material under the conditions of: at a reaction temperature of 400-600°C, preferably 420-580°C, at a reaction pressure of 0-0.3MPa, preferably 0.01-0.2MPa, and at a weight space velocity of 1-50h⁻¹, preferably 2-40h⁻¹.

As compared with the prior art, the invention may have the following advantages.

Provided in the present disclosure are an MFI molecular sieve, a particle catalyst containing the same, and an MFI molecular sieve catalyst containing the same. Because the ratio of contents of the monoclinic crystal form to the orthorhombic crystal form is in a predetermined range and the hydroxyl infrared spectrum of the catalyst shows a hydroxyl characteristic peak at 3400-3500cm⁻¹, the MFI molecular sieve catalyst in accordance with the present disclosure has high stability, high conversion rate for olefin raw materials, and high total selectivity for the products propylene and ethylene when used for producing propylene and ethylene via catalytic cracking. Moreover, the catalyst in accordance with the present disclosure has a specific surface area larger than those of the existing catalysts in the art, and thereby has a further improved conversion rate for olefin raw materials.

### Description of the drawings

Fig. 1 is an XRD spectrum of the catalyst obtained in Example 1;
Fig. 2 is a hydroxyl infrared spectrum of the catalyst obtained in Example 1;
Fig. 3 is an XRD spectrum of the catalyst obtained in Example 3;
Fig. 4 is an XRD spectrum of the catalyst obtained in Comparative Example 1;
Fig. 5 is a hydroxyl infrared spectrum of the catalyst obtained in Comparative Example 2.

### Detailed description

The invention will be further described below through the examples.

In the present disclosure, the specific surface area was measured on a TriStar 3000 physical adsorption instrument. After vacuum treatment at 300°C for 3 hours, the sample was placed in the instrument and liquid nitrogen was added, to operate the test. The Barret-Joyner-Halenda (BJH) model was used to calculate the specific surface area of the sample.

In the present disclosure, XRD analysis was operated on a Rigaku D/MAX-1400X polycrystalline X-ray diffractometer, under conditions of: **with a graphite** monochromator, Cu Kα rays, at a tube voltage of 40 kV, a tube current of 40 mA, and a scanning range 2θ of 2-70°. The XRD data was refined by a software, and the lattice constant (lattice-constant) was calculated, to obtain the ratio of the orthorhombic crystal form to the monoclinic crystal form of the catalyst.

In the present disclosure, the molar ratio of SiO₂/Al₂O₃ was calculated based on the elemental composition of the solid sample analyzed by using a Magix X-ray fluorescence spectrometer from Philips Company of the Netherlands, which was operated at a voltage of 40 kV and a current of 40 mA.

In the examples and comparative examples, the producing propylene and ethylene via catalytic cracking was operated by using at least one of C4-C6 olefins as a raw material, wherein: conversion rate for olefin raw materials (%) = (1-weight of olefins in the product/weight of olefins in the raw material) ×100%; selectivity for the two olefin products (%) = the total weight of propylene and ethylene obtained in the product/(weight of olefins in the raw material-weight of olefins remained after the reaction) × 100%.

In the present disclosure, the software for refining was Topas software, and the method for refining was the Rietveld whole pattern fitting method for crystal structural refinement. As known in the art, Rietveld refinement was mainly used to obtain information such as the crystalline structure and phase composition of a sample from its XRD spectrum. As an example, a model of an initial structure of the sample was first established (for example, using a known model from the molecular sieve structure database of the International Molecular Sieve Association). Then, by continuously adjusting parameters of the model (such as, atomic coordinates, temperature factors, unit cell parameters, peak parameters, background parameters, proportional factors, and the like, as known in the art), the XRD spectrum calculated from the initial structure gradually approached the actual spectrum. When the difference between the calculated spectrum and the actual spectrum was small to a certain degree (for example, Rwp < 10.0% as a convergence judgment criterion), it could be considered that the crystalline structure at this time was accurate, and the atomic coordinates, unit cell parameters, proportional factors and the like involved therein were the actual values, wherein the proportional factors represented the composition of various phases in the molecular sieve. Those skilled in the art could adjust and determine the particular refinements based on their professional knowledge and various factors of Rietveld refinement known in the art. The Rietveld refinement in the present disclosure was operated on GSAS software (see, for example, Larson, A.; Von Dreele, R. B. General Structure Analysis System GSAS; Los Alamos National Laboratory: Los Alamos, NM, 1996).

In the present disclosure, when calculating the peak area and its proportion of the hydroxyl infrared spectrum, the integrated area was concerned, and the line connecting the two lowest points in the characteristic peak curve of the spectrum was the starting reference line for the integration. After peak dividing, the area of the peaks was integrated, to obtain the peak area of the one centering around the position of the wave number of 3450 and having a half-peak width of 286 cm⁻¹, which was in a proportion of 86%. The hydroxyl characteristic peak had a highest point between 3420-3480 cm⁻¹. Integration was operated by centering around the wave number of the highest point and using the line connecting the two lowest points in the curve of the spectrum as the starting reference line, to calculate the peak area of the one having a half-peak width of 210-240 and the total area ofthe characteristic peak curve respectively, and then to calculate the proportion of the peak area ofthe one having the half-peak width of 210-240 to the total area.

### Example 1

### Step 1: Preparation of a molecular sieve raw powder

A silicon source, an aluminum source, templates T1 and T2 and water were mixed, to obtain a raw gel. The raw gel was fed into a supergravity integrated device through the feeding system of the supergravity integrated device in a total amount of 1L at a feed rate of 50mL/min. The silicon source was silica sol, the aluminum source was aluminum sulfate, the template T1 was TPABr, and T2 is phthalimide. Additionally added thereto were a boron group element R1 which was Ga and a nitrogen group element R2 which was P, wherein Ga source was GaCl₃, and P source was H₃PO₄. The silicon source, the aluminum source, the templates and water were in molar ratios of: H₂O/SiO₂=20; Si/Al=300; T1/SiO₂=0.05; R1/SiO₂=0.03; R2/SiO₂=0.02; and the ratio of T1 to T2 was adjusted to T2/T1=1.2. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain a ZSM-5 molecular sieve raw powder.

### Step 2: Preparation of a catalyst particle

100 g of the above ZSM-5 molecular sieve raw powder, 20 g of silica sol containing 40 wt% SiO₂ and 0.06 g of alumina were kneaded, extruded into strips, and dried at 80 °C for 10 h, to obtain the catalyst particle.

### Step 3: Preparation of a ZSM-5 molecular sieve

The obtained catalyst particles were placed in an atmosphere of template T3 which was ethylenediamine in a weight concentration of 10%, to operate a gas-solid phase hydrothermal crystallization at 130°C for 112 hours. The atmosphere of template T3 was generated by volatilizing an aqueous solution containing the template T3 ethylenediamine under autogenous pressure in a closed system. The aqueous solution containing the template T3 ethylenediamine and the catalyst particles were in a weight ratio of 1.5. After the hydrothermal crystallization, the product was removed, washed with distilled water, dried at 90°C for 15 hours, and then calcined at 550°C for 10 hours under an air atmosphere.

The calcined product was subjected to ammonium exchanging for three times in a 5 wt % ammonium nitrate solution at 90°C, drying, and calcining in a muffle furnace at 500°C for 4 h, to obtain a ZSM-5 molecular sieve catalyst.

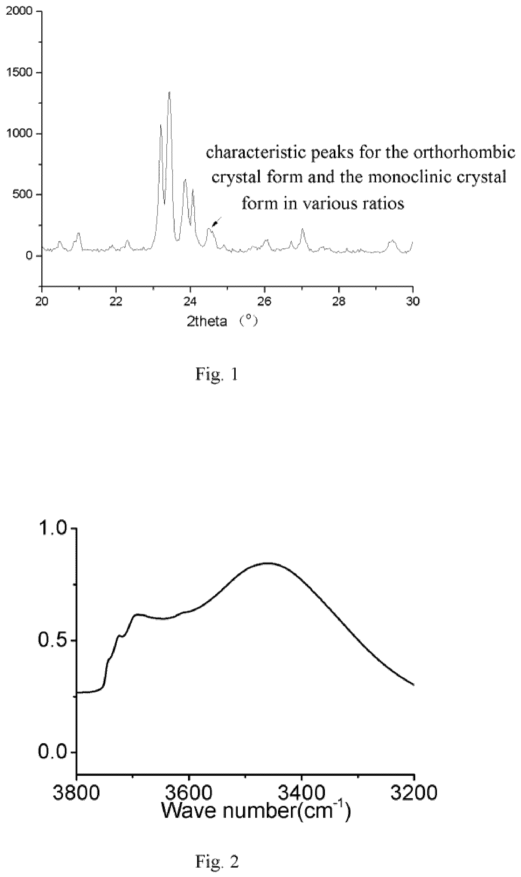

The obtained ZSM-5 molecular sieve catalyst was characterized by XRD, as shown in Fig. 1. The XRD data was refined by software, and the lattice constant (lattice-constant) was calculated. Results showed that the catalyst had the monoclinic crystal form and the orthorhombic crystal form in a ratio of 8:1, and a binder content of less than 0.2%. When characterizing the catalyst by nitrogen adsorption and desorption, the catalyst had a specific surface area of 420cm²/g. As analyzed by an X-ray fluorescence spectrometer, the catalyst had a molar ratio of SiO₂/Al₂O₃ of 298. In the obtained catalyst, the catalyst comprised ZSM-5 molecular sieve in a weight fraction of 98.9%, Ga element in a weight fraction of 0.5%, P element in a weight fraction of 0.5%, and a balance of the binder.

Fig. 2 was the hydroxyl infrared spectrum of the catalyst. The hydroxyl infrared spectrum of the catalyst showed hydroxyl characteristic peaks at 3400-3500 cm⁻¹. After peak dividing, the area of the peaks was integrated, to obtain the peak area of the one centering around the position of the wave number of 3450 and having a half-peak width of 286 cm⁻¹, which was in a proportion of 86%.

The obtained catalyst was evaluated on the activity for producing propylene and ethylene via catalytic cracking of olefins in a fixed bed catalytic reaction device by using mixed C4 (40 % by weight of butane and 60% by weight of butene) from an ethylene plant as raw materials. The evaluation was operated under conditions of: at a catalyst loading of 3 grams, a reaction temperature of 530°C, a reaction pressure of 0.03MPa, and a weight space velocity of 20h⁻¹. The results were: a conversion rate for C4 olefins of 79%, and a selectivity for propylene and ethylene of 81%. After operation for 80 hours over the catalyst, the activity and the selectivity of the catalyst did not substantially change, showing good stability.

### Example 2

### Step 1: Preparation of a molecular sieve raw powder

A silicon source, an aluminum source, templates T1 and T2 and water were mixed, to obtain a raw gel. The raw gel was fed into a supergravity integrated device through the feeding system thereof at a feed rate of 50mL/min. The silicon source was silica sol, the aluminum source was aluminum sulfate, the template T1 was TPABr, and T2 is phthalimide. The silicon source, the aluminum source, the templates and water were in molar ratios of: H₂O/SiO₂=20; Si/Al=300; T1/SiO₂=0.05; and the ratio of T1 to T2 was adjusted to T2/T1=1.2. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain a ZSM-5 molecular sieve raw powder.

### Step 2: Preparation of a catalyst particle

100 g of the above ZSM-5 molecular sieve raw powder, 20 g of silica sol containing 40 wt% SiO₂ and 0.06 g of alumina were kneaded, extruded into strips, and dried at 80 °C for 10 h, to obtain the catalyst particle.

### Step 3: Preparation of a ZSM-5 molecular sieve

The obtained catalyst particles were placed in an atmosphere of template T3 which was ethylenediamine in a weight concentration of 30%, to operate a gas-solid phase hydrothermal crystallization at 130°C for 112 hours. The atmosphere of template T3 was generated by volatilizing an aqueous solution containing the template T3 ethylenediamine under autogenous pressure in a closed system. The aqueous solution containing the template T3 ethylenediamine and the catalyst particles were in a weight ratio of 1.5. After the hydrothermal crystallization, the product was removed, washed with distilled water, dried at 90°C for 15 hours, and then calcined at 550°C for 10 hours under an air atmosphere.

The product was subjected to ammonium exchanging for three times in a 5 wt % ammonium nitrate solution at 90°C, drying, and calcining in a muffle furnace at 500°C for 4 h, to obtain a ZSM-5 molecular sieve catalyst.

The obtained ZSM-5 molecular sieve catalyst was characterized by XRD, the XRD data was refined by software, and the lattice constant (lattice-constant) was calculated. Results showed that the catalyst had the monoclinic crystal form and the orthorhombic crystal form in a ratio of 8:1, and a binder content of less than 0.2%. When characterizing the catalyst by nitrogen adsorption and desorption, the catalyst had a specific surface area of 410cm²/g. As analyzed by an X-ray fluorescence spectrometer, the catalyst had a molar ratio of SiO₂/Al₂O₃ of 304. In the obtained catalyst, the catalyst comprised ZSM-5 molecular sieve in a weight fraction of 99.9% and a balance of the binder.

The hydroxyl infrared spectrum of the catalyst showed hydroxyl characteristic peaks at 3400-3500 cm⁻¹. After peak dividing, the area of the peaks was integrated, to obtain the peak area of the one centering around the position of the wave number of 3448 and having a half-peak width of 272 cm⁻¹, which was in a proportion of 82%.

The obtained catalyst was evaluated on the activity for producing propylene and ethylene via catalytic cracking of olefins in a fixed bed catalytic reaction device by using mixed C4 (40 % by weight of butane and 60% by weight of butene) from an ethylene plant as raw materials. The evaluation was operated under conditions of: at a catalyst loading of 3 grams, a reaction temperature of 530°C, a reaction pressure of 0.03MPa, and a weight space velocity of 20h⁻¹. The results were: a conversion rate for C4 olefins of 78%, and a selectivity for propylene and ethylene of 80%. After operation for 80 hours over the catalyst, the activity and the selectivity of the catalyst did not substantially change, showing good stability.

### Example 3

### Step 1: Preparation of a molecular sieve raw powder

A silicon source, an aluminum source, templates T1 and T2 and water were mixed, to obtain a raw gel. The raw gel was fed into a supergravity integrated device through the feeding system thereof at a feed rate of 50mL/min. The silicon source was silica sol, the aluminum source was aluminum sulfate, the template T1 was TPABr, and T2 is naphthalene diamine. Additionally added thereto were a boron group element R1 which was Ga and a nitrogen group element R2 which was P, wherein Ga source was GaCl₃, and P source was H₃PO₄. The silicon source, the aluminum source, the templates and water were in molar ratios of: H₂O/SiO₂=20; Si/Al=300; T1/SiO₂=0.05; R1/SiO₂=0.03; R2/SiO₂=0.02; and the ratio of T1 to T2 was adjusted to T2/T1=3. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain a ZSM-5 molecular sieve raw powder.

### Step 2: Preparation of a catalyst particle

100 g of the above ZSM-5 molecular sieve raw powder, 20 g of silica sol containing 40 wt% SiO₂ and 0.06 g of alumina were kneaded, extruded into strips, and dried at 80 °C for 10 h, to obtain the catalyst particle.

### Step 3: Preparation of a ZSM-5 molecular sieve

The obtained catalyst particles were placed in an atmosphere of template T3 which was ethylenediamine in a weight concentration of 20%, to operate a gas-solid phase hydrothermal crystallization at 130°C for 112 hours. The atmosphere of template T3 was generated by volatilizing an aqueous solution containing the template T3 ethylenediamine under autogenous pressure in a closed system. The aqueous solution containing the template T3 ethylenediamine and the catalyst particles were in a weight ratio of 1.5. After the hydrothermal crystallization, the product was removed, washed with distilled water, dried at 90°C for 15 hours, and then calcined at 550°C for 10 hours under an air atmosphere.

The product was subjected to ammonium exchanging for three times in a 5 wt % ammonium nitrate solution at 90°C, drying, and calcining in a muffle furnace at 500°C for 4 h, to obtain a ZSM-5 molecular sieve catalyst.

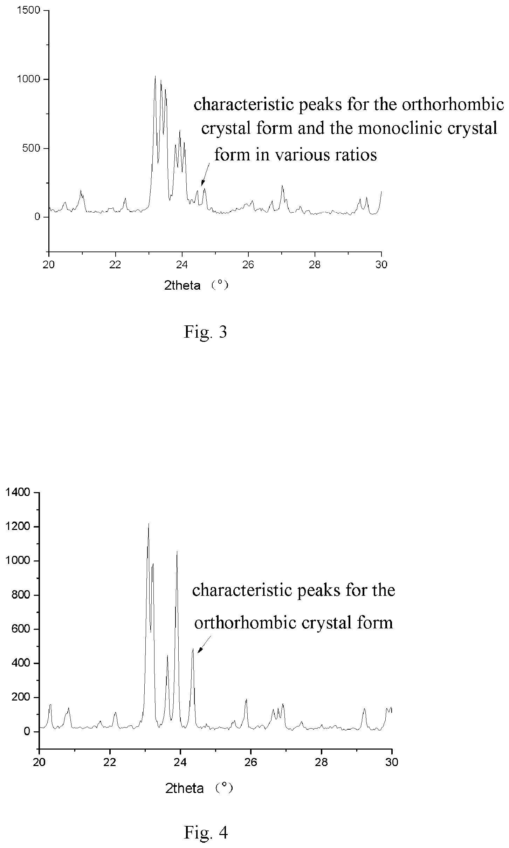

The obtained ZSM-5 molecular sieve catalyst was characterized by XRD, as shown in Fig. 3. The XRD data was refined by software, and the lattice constant (lattice-constant) was calculated. Results showed that the catalyst had the monoclinic crystal form and the orthorhombic crystal form in a ratio of 10:1, and a binder content of less than 0.2%. When characterizing the catalyst by nitrogen adsorption and desorption, the catalyst had a specific surface area of 400cm²/g. As analyzed by an X-ray fluorescence spectrometer, the catalyst had a molar ratio of SiO₂/Al₂O₃ of 297.

The hydroxyl infrared spectrum of the catalyst showed hydroxyl characteristic peaks at 3400-3500 cm⁻¹. After peak dividing, the area of the peaks was integrated, to obtain the peak area of the one centering around the position of the wave number of 3453 and having a half-peak width of 284 cm⁻¹, which was in a proportion of 84%.

In the obtained catalyst, the catalyst comprised ZSM-5 molecular sieve in a weight fraction of 98.8%, Ga element in a weight fraction of 0.6%, P element in a weight fraction of 0.5%, and a balance of the binder.
The obtained catalyst was evaluated on the activity for producing propylene and ethylene via catalytic cracking of olefins in a fixed bed catalytic reaction device by using mixed C4 (40 % by weight of butane and 60% by weight of butene) from an ethylene plant as raw materials. The evaluation was operated under conditions of: at a catalyst loading of 3 grams, a reaction temperature of 530°C, a reaction pressure of 0.03MPa, and a weight space velocity of 20h⁻¹. The results were: a conversion rate for C4 olefins of 77%, and a selectivity for propylene and ethylene of 79%. After operation for 80 hours over the catalyst, the activity and the selectivity of the catalyst did not substantially decrease.

### Comparative Example 1

### Step 1: Preparation of a molecular sieve raw powder

A silicon source, an aluminum source, a template T1 and water were mixed, to obtain a raw gel. The raw gel was fed into a supergravity integrated device through the feeding system thereof at a feed rate of 50mL/min. The silicon source was silica sol, the aluminum source was aluminum sulfate, the template T1 was TPABr. Additionally added thereto were a boron group element R1 which was Ga and a nitrogen group element R2 which was P, wherein Ga source was GaCl₃, and P source was H₃PO₄. The silicon source, the aluminum source, the template and water were in molar ratios of: H₂O/SiO₂=20; Si/Al=300; T1/SiO₂=0.11; R1/SiO₂=0.03; R2/SiO₂=0.02. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain a ZSM-5 molecular sieve raw powder.

### Step 2: Preparation of a catalyst particle

100 g of the above ZSM-5 molecular sieve raw powder, 20 g of silica sol containing 40 wt% SiO₂ and 0.06 g of alumina were kneaded, extruded into strips, and dried at 80 °C for 10 h, to obtain the catalyst particle.

### Step 3: Preparation of a ZSM-5 molecular sieve

The obtained catalyst particles were placed in an atmosphere of template T3 which was ethylenediamine in a weight concentration of 10%, to operate a gas-solid phase hydrothermal crystallization at 130°C for 112 hours. The atmosphere of template T3 was generated by volatilizing an aqueous solution containing the template T3 ethylenediamine under autogenous pressure in a closed system. The aqueous solution containing the template T3 ethylenediamine and the catalyst particles were in a weight ratio of 1.5. After the hydrothermal crystallization, the product was removed, washed with distilled water, dried at 90°C for 15 hours, and then calcined at 550°C for 10 hours under an air atmosphere.

The product was subjected to ammonium exchanging for three times in a 5 wt % ammonium nitrate solution at 90°C, drying, and calcining in a muffle furnace at 500°C for 4 h, to obtain a ZSM-5 molecular sieve catalyst.

The obtained ZSM-5 molecular sieve catalyst was characterized by XRD, as shown in Fig. 4. The XRD data was refined by software, and the lattice constant (lattice-constant) was calculated. Results showed that the catalyst had the orthorhombic crystal form in an amount of 100%, and a binder content of less than 0.2%. When characterizing the catalyst by nitrogen adsorption and desorption, the catalyst had a specific surface area of 200cm²/g. As analyzed by an X-ray fluorescence spectrometer, the catalyst had a molar ratio of SiO₂/Al₂O₃ of 296. In the obtained catalyst, the catalyst comprised ZSM-5 molecular sieve in a weight fraction of 98.9%, Ga element in a weight fraction of 0.5%, and P element in a weight fraction of 0.5%.

The hydroxyl infrared spectrum of the catalyst showed no hydroxyl characteristic peak at 3400-3500 cm⁻¹.

The obtained catalyst was evaluated on the activity for producing propylene and ethylene via catalytic cracking of olefins in a fixed bed catalytic reaction device by using mixed C4 (40 % by weight of butane and 60% by weight of butene) from an ethylene plant as raw materials. The evaluation was operated under conditions of: at a catalyst loading of 3 grams, a reaction temperature of 530°C, a reaction pressure of 0.03MPa, and a weight space velocity of 20h⁻¹. The results were: a conversion rate for C4 olefins of 59%, and a selectivity for propylene and ethylene of 60%. After operation for 80 hours over the catalyst, the activity and the selectivity of the catalyst substantially decreased.

### Comparative Example 2

### Step 1: Preparation of a molecular sieve raw powder

A silicon source, an aluminum source, a template T2 and water were mixed, to obtain a raw gel. The raw gel was fed into a supergravity integrated device through the feeding system thereof at a feed rate of 50mL/min. The silicon source was silica sol, the aluminum source was aluminum sulfate, and T2 is phthalimide. Additionally added thereto were a boron group element R1 which was Ga and a nitrogen group element R2 which was P, wherein Ga source was GaCl₃, and P source was H₃PO₄. The silicon source, the aluminum source, the template and water were in molar ratios of: H₂O/SiO₂=20; Si/Al=300; T2/SiO₂=0.11; R1/SiO₂=0.03; R2/SiO₂=0.02. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain an amorphous powder, failing in forming a molecular sieve catalyst.

### Comparative Example 3

### Step 1: Preparation of a molecular sieve raw powder

A silicon source, an aluminum source, a templates T1 and water were mixed, to obtain a raw gel. The raw gel was fed into a supergravity integrated device through the feeding system thereof at a feed rate of 50mL/min. The silicon source was silica sol, the aluminum source was aluminum sulfate, the template T1 was TPABr. Additionally added thereto were a boron group element R1 which was Ga and a nitrogen group element R2 which was P, wherein Ga source was GaCl₃, and P source was H₃PO₄. The silicon source, the aluminum source, the template and water were in molar ratios of: H₂O/SiO₂=20; Si/Al=300; T1/SiO₂=0.11; R1/SiO₂=0.03; R2/SiO₂=0.02. The raw gel was circulated in the reaction system of the supergravity aging-crystallizing integrated device, wherein the supergravity reactor had a rotation speed of 1500rpm, a flow rate of 100mL/min, and a residence time of 5h. This procedure made the gel mix evenly. The raw materials after the premixing and circulating were directly crystallized. After the once-through crystallization at 170°C for 12h in the supergravity reactor, the reaction was stopped. After cooling to room temperature, the product was removed out of the integrated device from the discharging system, and subjected to washing and centrifuging for three times, and drying at 80°C for 12 hours, to obtain a ZSM-5 molecular sieve raw powder.

### Step 2: Preparation of a catalyst particle

100 g of the above ZSM-5 molecular sieve raw powder, 20 g of silica sol containing 40 wt% SiO₂ and 0.06 g of alumina were kneaded, extruded into strips, and dried at 80 °C for 10 h, to obtain the catalyst particle.

### Step 3: Preparation of a ZSM-5 molecular sieve

The obtained catalyst particles were subjected to a conventional treatment with water vapor, a hydrothermal calcination at 550°C for 4 hours.

The product was subjected to ammonium exchanging for three times in a 5 wt % ammonium nitrate solution at 90°C, drying, and calcining in a muffle furnace at 500°C for 4 h, to obtain a ZSM-5 molecular sieve catalyst.

The obtained ZSM-5 molecular sieve catalyst was characterized by XRD, the XRD data was refined by software, and the lattice constant (lattice-constant) was calculated. Results showed that the catalyst had the monoclinic crystal form and the orthorhombic crystal form in a ratio of 50:1. When characterizing the catalyst by nitrogen adsorption and desorption, the catalyst had a specific surface area of 200cm²/g. As analyzed by an X-ray fluorescence spectrometer, the catalyst had a molar ratio of SiO₂/Al₂O₃ of 301. In the obtained catalyst, the catalyst comprised ZSM-5 molecular sieve in a weight fraction of 90%, Ga element in a weight fraction of 0.5%, and P element in a weight fraction of 0.5%.

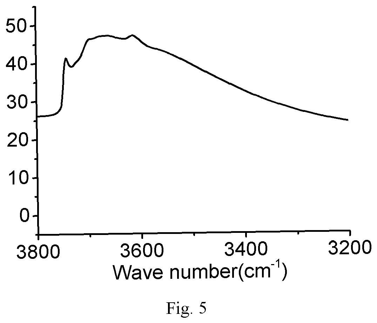

The hydroxyl infrared spectrum of the obtained catalyst was showed in Fig. 5. The hydroxyl infrared spectrum of the catalyst showed no hydroxyl characteristic peak at 3400-3500cm⁻¹.

The obtained catalyst was evaluated on the activity for producing propylene and ethylene via catalytic cracking of olefins in a fixed bed catalytic reaction device by using mixed C4 (40 % by weight of butane and 60% by weight of butene) from an ethylene plant as raw materials. The evaluation was operated under conditions of: at a catalyst loading of 3 grams, a reaction temperature of 530°C, a reaction pressure of 0.03MPa, and a weight space velocity of 20h⁻¹. The results were: a conversion rate for C4 olefins of 62%, and a selectivity for propylene and ethylene of 59%. After operation for 80 hours over the catalyst, the activity and the selectivity of the catalyst substantially decreased.

For ease of comparison, the main processing conditions and results were listed in Table 1.

**Table 1**

| | Templates used for synthesizing the molecular sieve raw powder | | | Properties of the catalysts | | | | Convent ion rate for C4 olefins, % | Selectivity for propylene and ethylene, % |
|---|---|---|---|---|---|---|---|---|---|
| | T1 | T2 | Molar ratio of T1/T2 | R1/weight fraction of R1 in the catalysts, % | R2/weight fraction of R2 in the catalysts, % | Molar ratio of monocli nic to orthorho mbic | Hydroxyl characteris tic peak at 3400-3500 cm⁻¹ | | |
| EX. 1 | TPABr | phthalimide | 1.2 | Ga/0.5 | P/0.5 | 8 | yes | 79 | 81 |
| EX. 2 | TPABr | phthalimide | 1.2 | none | none | 8 | yes | 75 | 77 |
| EX. 3 | TPABr | Naphthalene diamine | 3 | Ga/0.6 | P/0.5 | 10 | yes | 77 | 79 |
| CE. 1 | TPABr | -- | -- | Ga/0.5 | P/0.5 | 0 | no | 59 | 60 |
| CE. 2 | -- | phthalimide | 0 | -- | -- | -- | -- | -- | -- |
| CE. 3 | TPABr | -- | -- | Ga/0.5 | P/0.5 | 50 | no | 62 | 59 |

The embodiments of the present invention have been described in detail above. However, the present invention is not limited thereto. Various simple modifications may be made to the embodiments of the present invention within the technical scope of the present invention, including the combinations of various technical features in any other suitable way. Those simple modifications and combinations should also be regarded as the contents disclosed herein and being within the protection scope of the present disclosure.

## Claims

1. An MFI molecular sieve, **characterized in that**, each unit mass of the MFI molecular sieve comprises coexisting monoclinic crystal form and orthorhombic crystal form of the MFI molecular sieve; and the MFI molecular sieve shows hydroxyl characteristic peaks at 3400-3500 cm⁻¹ in the hydroxyl infrared spectrum thereof, wherein the characteristic peaks have the highest point at a position between 3420-3480 cm⁻¹, and the peak area of the one centering around the highest point and having a half-peak width of 210-240 is in a proportion of 70% or more of the total peak area; and wherein the MFI molecular sieve comprises the elements Si, Al and O, and is free of the element Ti.

2. The MFI molecular sieve of claim 1, **characterized in that**, each unit mass of the MFI molecular sieve comprises monoclinic crystal form and orthorhombic crystal form in a molar ratio of 5-100, preferably 6-50.

3. The MFI molecular sieve of claim 1, **characterized in that**, the MFI molecular sieve shows in the hydroxyl infrared spectrum thereof an absorption peak with the maximum intensity at 3460-3470 cm⁻¹.

4. The MFI molecular sieve of claim 1, **characterized in that**, the MFI molecular sieve has a molar ratio of SiO₂/Al₂O₃ of 80-1500.

5. A catalyst particle, comprising:
A) the MFI molecular sieve of any one of claims 1-3;
B) at least two different templates T1 and T2; and
C) a binder.

6. The catalyst particle of claim 5, **characterized in that**, the template T1 is at least one of tetrapropylammonium bromide, tetrapropylammonium hydroxide, tetramethylammonium bromide, tetraethylammonium bromide, tetrabutylammonium bromide, tetramethylammonium hydroxide, tetraethylammonium hydroxide, and tetrabutylammonium hydroxide; and/or, the template T2 is at least one of phthalimide, taurine, and naphthalene diamine.

7. The catalyst particle of claim 5, **characterized in that**, the binder is a silicon compound, or a mixture of a silicon compound and an aluminum compound; wherein the aluminum compound is at least one selected from the group consisting of alumina and aluminum sol, and the silicon compound is at least one selected from the group consisting of white carbon black and silica sol; and wherein, on the total weight of alumina and silica basis, the binder is added in an amount of 3%-50% of the total weight of the molecular sieve raw powder and the binder.

8. An MFI molecular sieve catalyst, comprising:
a) the MFI molecular sieve of any one of claims 1-3;
optionally, b) a boron group element R1; and
optionally, c) a nitrogen group element R2.

9. The MFI molecular sieve catalyst of claim 8, **characterized in that**, the MFI molecular sieve catalyst has a specific surface area of 200-1000 m²/g.

10. The MFI molecular sieve catalyst of claim 8, **characterized in that**, in the MFI molecular sieve catalyst, the molecular sieve is a hydrogen type molecular sieve.

11. The MFI molecular sieve catalyst of claim 8, **characterized in that**, based on the weight of the MFI molecular sieve catalyst, the MFI molecular sieve catalyst comprises:
a) the MFI molecular sieve, in an amount of 90-100%, preferably 92-99%;
b) the boron group element R1, in an amount of 0-5%, preferably 0.5-3.0%; and
c) the nitrogen group element R2, in an amount of 0-5%, preferably 0.5-5.0%.

12. The MFI molecular sieve catalyst of claim 8, **characterized in that**, in the MFI molecular sieve catalyst, the boron group element R1 is at least one selected from the group consisting of B and Ga; and/or, the nitrogen group element R2 is at least one selected from the group consisting of N, P, As, Sb and Bi.

13. The MFI molecular sieve catalyst of claim 8, **characterized in that**, the MFI molecular sieve catalyst further comprises a binder; and based on the weight of the catalyst, the binder is in an amount of 5% by weight or less.

14. The MFI molecular sieve catalyst of any one of claims 8-13, **characterized in that**, the MFI molecular sieve catalyst is obtained by subjecting the catalyst particles of any one of claims 5-7 to treatments comprising hydrothermal crystallization under an atmosphere of a template T3.

15. A method for preparing the MFI molecular sieve catalyst of any one of claims 8-14, comprising steps of:
Step 1: preparing an MFI molecular sieve raw powder;
Step 2: mixing the molecular sieve raw powder obtained in Step 1 with a binder, and optionally adding water, then subjected to kneading, shaping and drying, to obtain catalyst particles;
Step 3: subjecting the catalyst particles obtained in Step 2 to hydrothermal crystallization under an atmosphere of a template T3, then subjecting to a first calcining, ammonium exchanging and a second calcining, to obtain the MFI molecular sieve catalyst.

16. The method of claim 15, **characterized in that**, at least two different templates T1 and T2 are used in Step 1; preferably, T1 and T2 are in a molar ratio of T2/T1=0.01-30.

17. The method of claim 16, **characterized in that**, Step 1 comprises: preparing a raw gel, by mixing a silicon source, an aluminum source, the template T1, the template T2, water and optionally, a boron group element R1 and/or optionally, a nitrogen group element R2;
wherein the silicon source, the aluminum source, the templates, the boron group element, the nitrogen group element and water are in molar ratios of: H₂O/SiO₂=10-500; Si/Al=20-∞; T1/SiO₂=0.01-20; R1/SiO₂=0-50; R2/SiO₂=0-50.

18. The method of claim 16, **characterized in that**, the template T1 is at least one of tetrapropylammonium bromide, tetrapropylammonium hydroxide, tetramethylammonium bromide, tetraethylammonium bromide, tetrabutylammonium bromide, tetramethylammonium hydroxide, tetraethylammonium hydroxide, and tetrabutylammonium hydroxide; and/or, the template T2 is at least one of phthalimide, taurine, and naphthalene diamine.

19. The method of claim 15, **characterized in that**, in Step 2, the binder is a silicon compound, or a mixture of a silicon compound and an aluminum compound; wherein the aluminum compound is at least one selected from the group consisting of alumina and aluminum sol, and the silicon compound is at least one selected from the group consisting of white carbon black and silica sol; and wherein, on the total weight of alumina and silica basis, the binder is added in an amount of 3%-50% of the total weight of the molecular sieve raw powder and the binder.

20. The method of claim 15, **characterized in that**, in Step 3, the template T3 is at least one of aqueous ammonia, ethylamine, ethylenediamine, triethylamine, n-butylamine, hexamethylenediamine, tetrapropylammonium bromide or tetrapropylammonium hydroxide; the atmosphere of the template is generated by volatilizing an aqueous solution of the template T3 under autogenous pressure in a closed system; the aqueous solution of the template T3 has a weight concentration of 0.5-40%, and the aqueous solution of the template T3 and the catalyst particles are in a weight ratio of 1-5.

21. The method of claim 15, **characterized in that**, in Step 3, after hydrothermal crystallization, the first calcining is operated under conditions of: at a calcination temperature of 500-650°C, preferably 530-600°C, for a calcination time of 8-15h, preferably 10-12h; and/or, after ammonium exchanging, the second calcining is operated under conditions of: at a calcination temperature of 500-600°C for a calcination time of 4-8h.

22. The method of claim 15, **characterized in that**, in Step 3, after hydrothermal crystallization and before the first calcining, drying is operated, wherein the drying is operated under conditions of: at a drying temperature of 80-100°C, preferably 90-100°C, for a drying time of 10-20h, preferably 12-16h.

23. Use of the MFI molecular sieve catalyst of any one of claims 8-14 in producing propylene and ethylene via catalytic cracking of olefins.

24. A method for producing propylene and ethylene via catalytic cracking of olefins, comprising:
contacting an olefin raw material with the MFI molecular sieve catalyst of any one of claims 8-14 to react, to obtain the products propylene and ethylene,
wherein it is operated by using at least one of C4-C6 olefins as the raw material under the conditions of: at a reaction temperature of 400-600°C, at a reaction pressure of 0-0.3MPa, and at a weight space velocity of 1-50h⁻¹.
